# EUROPEAN PATENT APPLICATION

(11) **EP 2 839 821 A1**
(43) Date of publication of application: **25.02.2015**
(21) Application number: 13872271.5
(22) Date of filing: 29.10.2013
(51) Int. Cl.: A61H 1/00

(54) **FEELING REHABILITATION TRAINING SYSTEM AND IMPLEMENTATION METHOD THEREOF**

(30) Priority: 16.05.2013 CN 201310181540
(71) Applicant: Sichuan Xukang Medical Electrical Equipmentco.,Ltd, Chengdu, Sichuan 611730 (CN)
(72) Inventor: LI, Tong, Chengdu Sichuan 610000 (CN)
(74) Representative: Cabinet Chaillot
(86) International application number: PCT/CN2013/001303
(87) International publication number: WO 2014/183239

(57) **Abstract**

Exemplary systems and methods for providing a sensation rehabilitation training system and its implementation which solves the problems of existing sensation rehabilitation training device like large size, poor training results and high cost. The rehabilitation training system include the CPU as well as sensation training device (STD), pressure sensation training device (PSTD) and feedback device (FD) separately connected with the CPU, where the STD and PSTD are connected. The invention is characterized in rational design, high integration, brand-new training mode, wide range of application, easy operation and significant training effect, thus it is appropriate for promotion and application.

## Description

### Field of the Invention

The present invention relates to a training system, specifically, a sensation rehabilitation training system and its implementation methods.

### Background of the Invention

The sensation rehabilitation training is an essential sensation rehabilitation program for patients suffering loss of some or all sensations. With the help of rehabilitation training devices, the patients receive the systematic training to body parts in which the sensation is lost, and recovers sensation gradually. This is the more common way of rehabilitation. However, the current sensation rehabilitation training devices are bulky that some body parts (such as wrist, leg, back and waist) of the patients cannot comprehensively receive the sensation rehabilitation training. As a result, the optimal training effect may not be obtained, leading to a slow rehabilitation of sensation. Moreover, bulky device with complicated structure and operation also increases the rehabilitation cost. Thus, one of the urgent problems facing the experts in the field of sports rehabilitation is to design a small, low-cost sensation rehabilitation training device capable of delivering good training results.

### Summary of the Invention

The invention aims to provide a sensation rehabilitation training system and its implementation methods, which can solve the problems of current sensation rehabilitation training device, specifically, the large size, poor training effect and high cost.

To achieve the above objective, the invention applies the technical solutions as stated below:

A sensation rehabilitation training system is characterized in that it is composed of central processing unit (CPU) as well as sensation training device (STD), pressure sensation training device (PSTD) and feedback device (FD) separately connected with the CPU, where the PSTD and the STD are linked together.

As preferred, the feedback device is connected with the CPU with/without wire.

To be specific, the PSTD includes pressure bandage, pressure senor which is set in the bandage and electrically connected with the CPU, and micro air pump connected between the CPU and pressure bandage. The PSTD is built into the pressure bandage.

Further, the STD includes protective pad set in the pressure bandage and fixedly equipped with electrodes, temperature sensors and vibrating motor, and cold-hot transducer mounted on the motor; the electrode, vibrating motor, cold-hot transducer and temperature sensor are linked to the CPU.

Still further, the vibrating motor is flat motor, micro DC motor or micro stepper motor.

To be specific, the cold-hot transducer includes the carrier connected with the CPU, and hot wire and/or semiconductor refrigeration chip which are/is installed in the carrier.

As preferred, there are 16 electrodes which are distributed in rectangular matrix on the protective pad.

With the fore-mentioned hardware basics, the present invention also provides following two implementation methods for the sensation rehabilitation training system:
1. One method at least includes one of the following modes:
   Touch sensation training mode
      (1) Fix the pressure bandage onto the body part where rehabilitation training is required;
      (2) The CPU controls, according to the training solution, the electrode to glimmer and produce variously shaped stimulation;
      (3) The patient feeds back his actual feeling of the shaped stimulation to the CPU via the feedback device;
      (4) The CPU shows the results to the doctor who then revises the solution based on comparison of the patient's feedback and shaped stimulation actually given and sends the revision back to the CPU;
      (5) Steps (2) ∼ (4) are repeated till the patient is fully recovered.
   Temperature sensation training mode
      (1) Fix the pressure bandage onto the body part where rehabilitation training is required;
      (2) The CPU controls, according to the training solution, the cold/hot stimulations of different levels produced by the cold-hot transducer and transfers the stimulations onto the patient's skin;
      (3) The temperature sensor collects the real-time temperature on the skin surface which is then fed back to the CPU.
      (4) The CPU estimates the compliance of actual temperature to the solution, or else the cold-hot transducer under the control of the CPU gives new cold/hot stimulations onto the skin until the compliance is realized.
      (5) When the given temperature complies with the solution, the patient feeds his feeling of temperature back to the doctor who then revises the solution based on comparison of the patient's feedback and cold/hot stimulations actually given and sends the revision back to the CPU;
      (6) Steps (2) ∼ (5) are repeated till the patient is fully recovered.
2. The other method at least includes one of the following modes:
   Vibration sensation training mode
      (1) Fix the pressure bandage onto the body part where rehabilitation training is required;
      (2) The CPU controls, according to the training solution, the vibrating motor to produce vibrating stimulations of different levels which are transferred onto the patient's skin through pressure bandage;
      (3) The patient feeds back his actual feeling of the vibrating stimulation to the CPU via the feedback device;
      (4) The CPU shows the results to the doctor who then revises the solution based on comparison of the patient's feedback and vibrating stimulation actually given and sends the revision back to the CPU;
      (5) Steps (2) ∼ (4) are repeated till the patient is fully recovered.

### Pressure sensation training mode

(1) Fix the pressure bandage onto the body part where rehabilitation training is required;
(2) The CPU regulates, according to the training solution, the air pressure in the pressure bandage via micro air pump, and then the pressure sensor collects pressure between the patient's skin surface and the bandage which is fed back to the CPU;
(3) The CPU estimates the compliance of actual pressure to the solution, or else the micro air pump under the control of the CPU reregulates the air pressure in the bandage until the compliance is realized.
(4) When the given pressure complies with the solution, the patient feeds his feeling of pressure back to the doctor who then revises the solution based on comparison of the patient's feedback and pressure actually given and sends the revision back to the CPU;
(5) Steps (2), (3) and (4) are repeated till the patient is fully recovered.

Comparing the current technology, the invention is to provide the following beneficial effects.
1) The invention is designed into a simple structure with low cost and easy operation.
2) The invention is to realize local sensation rehabilitation by using the CPU to give command, the STD and PSTD to train the patients, and the feedback device to give feedback. As the electrodes of the sensation training device is arranged in rectangular array, different shapes of stimulations can be produced onto the patient skin when all or partial electrodes glimmer, and the doctor can make real-time adjustment to the training solution based on feedback of the patient, providing the patient with better training of touch sensing.
3) The invention provides the electrodes in the STD to produce cold and hot stimulations to the body part as needed with the temperatures from the cold-hot transducer and allows the doctor to make real-time adjustment to the training solution based on feedback of the patient, providing the patient with better training of temperature sensing. In addition, the temperature sensor provided herein is able to detect the temperature given onto the skin and send it to the CPU for comparing to the solution. This is helpful to reduce deviation from the solution during the implementation, ensuring more accurate implementation of the present invention.
4) The invention provides the patient with vibrating stimulations of different levels on the needy body parts as the CPU controls the vibration amplitude of the motor and allows the doctor to make real-time adjustment to the training solution based on feedback of the patient, providing the patient with better training of vibration sensing.
5) The invention provides the micro air pump in the PSTD, which under the control of the CPU, inflates the pressure bandage to tightly hold the body part to be trained, and allows the doctor to well know the patient's perception of pressure based on feedback of the patient, providing the patient with better training of pressure sensing. During the training, the pressure sensor feeds back the pressure given to the CPU, ensuring the pressure is given according to the training solution. This is helpful to reduce deviation from the solution, ensuring more accurate implementation of the invention.
6)The four modes of the invention are implemented by using the micro air pump under the control of the CPU to inflate the pressure bandage onto the body parts requiring training, where the operation is simple and flexible with good fixed effect.
7)The four modes of the invention functions independently and individually that the patient can chose any one of them to train the body part as needed for full recovery of local sensation. The invention pushes the current technical restriction and elaborately integrates the four training modes in a small, low-cost and easily-maintained system. Thus, the invention featuring the highly targeted, brand-new training modes, wide range of application, low cost and significant effect, prevails with the outstanding substantial characteristics and prominent progress when comparing to the existing technologies.
8) The invention reflects high practical and promotional values as it is of high performance cost ratio, well targeted and easy to be batch produced.

### Brief Description of the Drawings

Drawing 1: Front View of the Invention
Drawing 2: Bottom View of the Invention
Where, name of parts and components is respectively indicated on the drawing:
1-CPU; 2-Pressure bandage; 3-Protective pad; 4-Electrode; 5-Vibrating motor; 6-Cold-hot transducer; 7-Temperature sensor; 8-Pressure senor; 9-Micro air pump; 10-Feedback device.

### Detailed Description of the Preferred Embodiments

The further instruction of the invention is given by combining drawings and embodiments below. The implementation method of the invention includes but not limited to the following embodiments.

### Embodiment 1

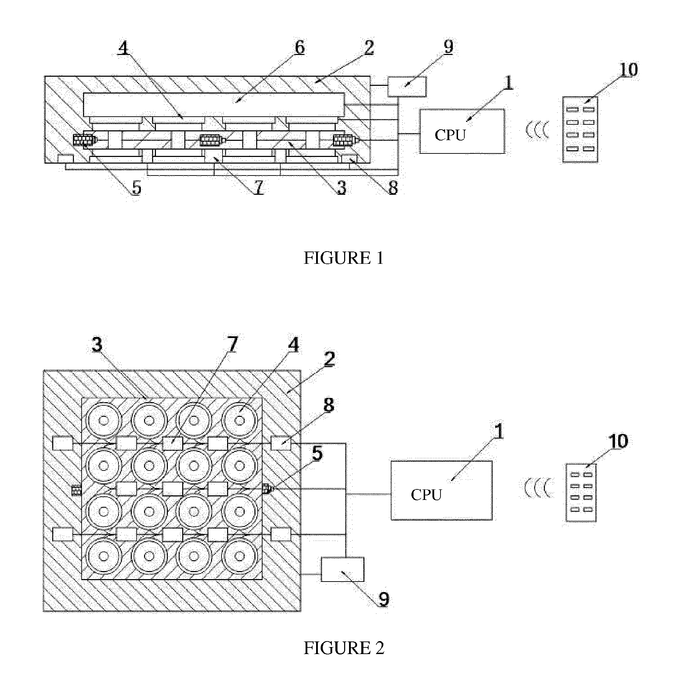

As shown in Drawing 1 and 2, the invention includes the CPU, the sensation training device (STD), the pressure sensation training device (PSTD) and feedback device. The STD, PSTD and feedback device are linked with the CPU, where the CPU functions, as the same as the current controllers, to control other components working; the STD and PSTD are used to train and recover the sensation of the patient's local parts such as wrist, arm, leg and back; and the feedback device is to send back the patient's feedback information during the training, and it is connected with the CPU with/without wire.

To be specific, the PSTD includes pressure bandage, pressure senor which is set in the bandage and electrically connected with the CPU, and micro air pump connected between the CPU and pressure bandage. The STD includes protective pad set in the pressure bandage and fixedly equipped with electrodes, temperature sensors, vibrating motor and cold-hot transducer mounted on the motor; the electrode, vibrating motor, cold-hot transducer and temperature sensor are linked to the CPU. The electrode, vibrating motor, cold-hot transducer, temperature sensor, pressure sensor and micro air pump operate under the control of the CPU. In this embodiment as preferred, there are 16 electrodes distributed in rectangular matrix on the protective pad, and further, the electrode is made of conductive silicone or metal.

The cold-hot transducer can produce cold and hot stimulations of different levels and it includes the carrier connected with the CPU, and hot wire and/or semiconductor refrigeration chip which are/is detachably mounted in the carrier. The patient can install hot wire and/or semiconductor refrigeration chip for hot and/or cold simulation according to the need. In addition, the hot wire and/or semiconductor refrigeration chip can also be mounted in the carrier in the factory. In this embodiment as preferred, the cold-hot transducer is provided with both hot wire and semiconductor refrigeration chip which are detachably mounted in the carrier.

Furthermore, the invention is provided with nine pressure sensors to accurately collect temperatures on the skin surface, and three vibrating motors and four pressure sensors.

With the foresaid hardware basics, the invention realizes four working modes including touch sensation training mode, temperature sensation training mode, vibration sensation training mode and pressure sensation training mode, providing rehabilitation training of touch, pressure, vibrating and pressure sensations to the patient. The implementation is detailed as follows:

### (1) Touch sensation training mode

When using, the micro air pump under the control of the CPU inflates the pressure bandage to tightly hold the body part of the patient. The protective pad made of soft materials can be bent, making the electrode closely against the skin, and the doctor has the CPU to control all or some electrodes glimmering, producing stimulations of various shapes to the patient. For instance, it provides square stimulation when all electrodes glimmer, and "--" shaped, triangle, or "L" shaped stimulation when some electrodes on the different positions glimmer. The feedback device, as the same as the ordinary remote control, is composed of remote control panel and multiple keys thereon, one key for one shape. When receiving stimulation, the patient tells shape of the stimulation through self-perception, and presses the appropriate key on the feedback device according to the sensing so as to feed the information back to the CPU. In the embodiment, information transmission between the feedback and the CPU is realized via wireless communication technology. And of course, the infrared technology is applicable.

When receiving feedback information, the CPU works on conventional signal processing and shows the results to the doctor who then can judge whether the patient has his touch sensing ability recovered by comparing the shape of stimulation told by the patient with that actually given. Agreement between the feedback and the given indicates the complete rehabilitation of touch sensing ability, and the training can be terminated, on the contrary, the doctor will make adjustment to the training solution based on the feedback before continuing to provide training to the patient. The process will repeat until the patient has his ability of touch sensing fully recovered.

### (2) Temperature sensation training mode

When using, the micro air pump under the control of the CPU inflates the pressure bandage to tightly hold the body part of the patient. The doctor starts the cold-hot transducer under the control of the CPU and to produce different cold and hot stimulations which are transferred to the patient on the training part. During the process, the pressure sensor collects real-time temperature on the patient's skin surface, and sends the data back to the CPU which then estimates the compliance of actual temperature to the solution. When the compliance is realized, hot and cold stimulations are stopped and the patient tells the doctor about temperature sensed. The doctor compares the feedback information with the cold and hot stimulations produced by the transducer and judge the patient's ability of temperature sensing. As similar to the touch sensation training mode, training will be terminated if the comparison indicates the patient has his ability of temperature sensing recovered. On the contrary, the doctor will make adjustment to the training solution before continuing to provide training to the patient. The process will repeat until the patient has his ability of temperature sensing recovered fully recovered.

### (3) Vibrating sensation training mode

When using, the micro air pump under the control of the CPU inflates the pressure bandage to tightly hold the body part of the patient. The doctor starts the vibrating motor to vibrate under the control of the CPU. The vibration amplitude of the motor is adjustable under the control of the CPU to produce vibrating stimulation of different levels. In addition to keys for the touch sensation training mode, the feedback device is also provided with keys for vibrating sensation training mode at high, medium, low and neutral level of stimulation. When receiving the vibrating stimulation, the patient presses the related keys on the feedback device based on his actual feeling and the feedback information is then sent back to the CPU. The CPU works on conventional signal processing and shows the results to the doctor who then can judge whether the patient has his ability of vibrating sensing recovered by comparing the intensity of vibrating stimulation told by the patient with that actually given. Agreement between the feedback and the given indicates the complete rehabilitation of touch sensing ability, and the training can be terminated, on the contrary, the doctor will make adjustment to the training solution before continuing to provide training to the patient. The process will repeat until the patient has his ability fully recovered.

### (4) Pressure sensation training mode

When using, the micro air pump under the control of the CPU inflates the pressure bandage to tightly hold the body part of the patient and the pressure sensor is also closely against the skin. Air pressure in the pressure bandage can be regulated by controlling inflating volume of the micro air pump. During the process, the pressure sensor collects real-time pressure between the patient's skin and the pressure bandage, and sends data back to the CPU. The CPU then works on the data processing and estimates the compliance of the given pressure to the solution. When the compliance is realized, inflating is stopped. The doctor compares the feedback information with pressure given by the pressure bandage and judges the patient's ability of pressure sensing. Eventually, more specific pressure sensation training solution will be developed by the doctor and provided to the patient. The process will repeat until the patient has his ability of pressure sensing recovered fully recovered.

It's worth noting that the feedback device of the invention may be varied in types as it can have fixed structure and shape, or can be a virtual mode of signal transmission where the patient directly tells the doctor about his feeling or records his oral statement and remotely transmit it to the doctor. In the touch and vibrating sensation training modes of the invention, a prior consideration is given to the feedback device with fixed structure such as a computer which allows the patient to feed back his actual feeling to the doctor via computer internet, or a mobile phone which allows feeding back via mobile network. While as for the temperature and pressure sensation training modes, the mode of signal transmission is preferred for feedback of patient's feeling, for example, the patient directly tells the doctor his feeling. Of course, the feedback mode is unrestricted in the invention as it has no significant impact on the training results. Thus, application of any feedback mode remains within the scope of protection of the invention.

According to the embodiment above, the invention can be well implemented. It is noted that to solve the same technical problem, some non-substantive modifications or polishing may be made to the invention based on the foresaid structural design, even so, the technical solution applied remains the same essence with the invention and should also belong to the scope of protection of the invention.

## Claims

1. A system comprising: a central processing unit (CPU) as well as a sensation training device (STD), a pressure sensation training device (PSTD) and a feedback device separately connected with the CPU, where the PSTD includes a pressure bandage, a pressure senor which is set in the bandage and electrically connected with the CPU, and a micro air pump which is electrically connected with the CPU and used to connect the pressure bandage; the PSTD is built into the pressure bandage and connected with the CPU; the STD includes a protective pad set in the pressure bandage and fixedly equipped with an electrodes, a temperature sensors, a vibrating motor, and a cold-hot transducer mounted on a motor; a electrode, a vibrating motor, a cold-hot transducer and a temperature sensor are linked to the CPU.

2. The system of claim 1, wherein: the cold-hot transducer includes a carrier connected with the CPU, and a hot wire or/and a semiconductor refrigeration chip which is/are installed in a carrier.

3. The system of claim 2, wherein: there are 16 electrodes which are distributed in rectangular matrix on the protective pad.

4. The system of claim 3, wherein: a vibrating motor is a flat motor, a micro DC motor or a micro stepper motor.

5. The system of claim 4, wherein: the feedback device is linked with the CPU with/without wire.

6. A method comprising: providing at least any of the following two modes:
Touch sensation training mode
(1) fix the pressure bandage onto the body part where rehabilitation training is required;
(2) a CPU controls, according to a training solution, a electrode to glimmer and produce some variously shaped stimulation;
(3) the patient feeds back his actual feeling of the shaped stimulation to the CPU via the feedback device;
(4) the CPU shows the results to the doctor who then revises the solution based on comparison of the patient's feedback and shaped stimulation actually given and sends the revision back to the CPU;
(5) steps (2) ∼ (4) are repeated till the patient is fully recovered;
Temperature sensation training mode
(1) fix the pressure bandage onto the body part where rehabilitation training is required;
(2) the CPU controls, according to a training solution, a cold/hot stimulations of different levels produced by a cold-hot transducer and a transfers the stimulations onto a patient's skin;
(3) a temperature sensor collects a real-time temperature on the skin surface which is then fed back to the CPU;
(4) the CPU estimates the compliance of actual temperature to the solution, or else the cold-hot transducer under the control of the CPU gives a new cold/hot stimulations onto the skin until the compliance is realized;
(5) when the given temperature complies with the solution, the patient feeds his feeling of temperature back to the doctor who then revises the solution based on comparison of a patient's feedback and a cold/hot stimulations actually given and sends a revision back to the CPU;
(6) steps (2) ∼ (5) are repeated till the patient is fully recovered.

7. A method comprising: providing at least any of the following two modes:
Vibration sensation training mode
(1) fix the pressure bandage onto the body part where a rehabilitation training is required;
(2) the CPU controls, according to a training solution, the vibrating motor to produce vibrating stimulations of different levels which are transferred onto the patient's skin through pressure bandage;
(3) the patient feeds back his actual feeling of a vibrating stimulation to the CPU via the feedback device;
(4) the CPU shows the results to the doctor who then revises the solution based on comparison of a patient's feedback and a vibrating stimulation actually given and sends a revision back to the CPU;
(5) steps (2) ∼ (4) are repeated till the patient is fully recovered;
Pressure sensation training mode
(1) fix the pressure bandage onto the body part where rehabilitation training is required;
(2) the CPU regulates, according to the training solution, a air pressure in a pressure bandage via a micro air pump, and then a pressure sensor collects pressure between a patient's skin surface and a bandage which is fed back to the CPU;
(3) the CPU estimates the compliance of actual pressure to the solution, or else a micro air pump under a control of the CPU reregulates a air pressure in the bandage until the compliance is realized;
(4) when the given pressure complies with the solution, the patient feeds his feeling of pressure back to the doctor who then revises the solution based on comparison of the patient's feedback and pressure actually given and sends a revision back to the CPU;
(5) steps (2) ∼ (4) are repeated till the patient is fully recovered.
